# EUROPEAN PATENT APPLICATION

(11) **EP 4 807 012 A1**
(43) Date of publication of application: **16.09.2026**
(21) Application number: 23958156.4
(22) Date of filing: 19.12.2023
(51) Int. Cl.: C12P 7/44

(54) **METHOD FOR PRODUCING LONG-CHAIN DICARBOXYLIC ACID AND LONG-CHAIN DICARBOXYLIC ACID**

(30) Priority: 08.11.2023 CN 202311479508
(71) Applicant: Cathay Biotech Inc., Shanghai 201203 (CN); CIBT America Inc., Newark, DE 19713 (US); Cathay (Jinxiang) Biomaterial Co., Ltd., Jining, Shandong 272211 (CN)
(72) Inventor: HAO, Yingli, Shanghai 201203 (CN); LV, Huan, Shanghai 201203 (CN); XU, Min, Shanghai 201203 (CN); LIU, Xiucai, Shanghai 201203 (CN)
(74) Representative: SSM Sandmair
(86) International application number: PCT/CN2023/139802
(87) International publication number: WO 2025/097558

(57) **Abstract**

Provided are a method for producing a long-chain dicarboxylic acid and a long-chain dicarboxylic acid. The method for producing the long-chain dicarboxylic acid comprises: inoculating a seed solution of a fermentation strain into a fermentation culture medium and culturing, and adding a substrate fatty acid ester and fermenting. The fatty acid ester is used as a substrate, which can replace fossil fuels such as petroleum alkanes; a long-chain dicarboxylic acid having 100% bio-based content and 98% or greater purity is synthesized, and the present invention has a high yield and conversion rate.

## Description

### TECHNICAL FIELD

The present disclosure pertains to the technical field of fermentation, and particularly relates to a method for producing a long-chain dicarboxylic acid and a long-chain dicarboxylic acid.

### BACKGROUND

Long-chain dibasic acids, also known as long-chain dicarboxylic acids, refer to linear aliphatic dicarboxylic acids containing 10 or more carbon atoms in the carbon chain. They can be used not only as monomers for synthesizing high-performance polyamides, but also as important raw materials for fragrances, coatings, lubricants, paints, plasticizers, new pharmaceuticals, pesticides, and the like. Long-chain dicarboxylic acids can serve as raw materials for synthesizing nylon 6-12 and molding resins, modifiers for saturated polyesters, as well as raw materials for powder coatings, plasticizers, synthetic lubricants, metal precipitants, and synthetic macrocyclic musk.

Long-chain dicarboxylic acids have important and widespread industrial uses, but they do not exist independently in nature, and their sources vary depending on the chain length. Synthesis methods include chemical synthesis methods and biological fermentation methods. Chemical synthesis methods are characterized by complex processes, harsh conditions, many steps, low yields, low purity, high costs, pollution, and the like. Biological methods typically use corresponding long-chain alkanes and derivatives thereof as substrates to produce long-chain dicarboxylic acids by fermentation using *Candida tropicalis* or *Candida viswanathii*. Compared with chemical synthesis methods, biological methods are characterized by simple production processes, mild conditions, specific reactions, higher yields, low energy consumption, and simple extraction and purification processes.

Research work on long-chain dicarboxylic acids has been conducted for more than 40 years in China, mainly using long-chain alkanes as substrates. Fatty acid esters can be obtained by esterification of fatty acids with various alcohols. Such novel renewable resources can replace fossil raw materials such as petroleum alkanes, which is of great significance for the sustainable development of industrial processes.

### SUMMARY OF THE INVENTION

In one aspect of the present disclosure, the present disclosure provides a method for producing a long-chain dicarboxylic acid, and the production method uses a fatty acid ester as a substrate to produce a long-chain dicarboxylic acid by fermentation, so as to replace fossil fuels such as petroleum alkanes. The target product long-chain dicarboxylic acid has a bio-based content of 100% and a purity higher than 98%, and has relatively high yield and conversion rate.

In order to achieve the above objective, the present disclosure provides a method for producing a long-chain dicarboxylic acid by fermentation, and the method comprises: inoculating a seed liquid of a fermentation strain into a fermentation culture medium and culturing, and adding a substrate fatty acid ester to perform fermentation. Specifically, during the fermentation, a carbon source is supplemented into the fermentation tank to ensure sufficient energy for bacterial cells. The addition mode of the carbon source may be batch supplementation or continuous feeding.

In some embodiments, the method comprises the following steps:
(1) subjecting the fermentation strain to expansion culture until the seed liquid of the fermentation strain has an optical density value OD₆₂₀ of 0.5-1.0 after a 30-fold dilution;
(2) inoculating the seed liquid into a fermentation culture medium, and when the fermentation broth has an optical density value OD₆₂₀ of 0.5-1.0 after a 30-fold dilution, starting supplementing the fatty acid ester to obtain a long-chain dicarboxylic acid fermentation broth; and
(3) purifying the long-chain dicarboxylic acid fermentation broth to obtain a long-chain dicarboxylic acid.

In the present disclosure, the long-chain dicarboxylic acid has a chemical formula of HOOC(CH₂)nCOOH, wherein n ≥ 8; the long-chain dicarboxylic acid comprises any one of decanedioic acid, undecanedioic acid, dodecanedioic acid, tridecanedioic acid, tetradecanedioic acid, pentadecanedioic acid, hexadecanedioic acid, heptadecanedioic acid, and octadecanedioic acid.

In the present disclosure, the carbon source comprises any one or two of glucose, fructose, sucrose, and maltose.

In the present disclosure, the fermentation strain is not particularly limited and comprises *Candida viswanathii*, *Candida albicans*, *Candida tropicalis*, *Candida sake*, or *Yarrowia lipolytica*.

In the present disclosure, the fatty acid ester is not particularly limited and comprises any one of a decanoate, an undecanoate, a laurate (i.e., a dodecanoate), a tridecanoate, a myristate (i.e., a tetradecanoate), a pentadecanoate, a palmitate (i.e., a hexadecanoate), a heptadecanoate, and an octadecanoate, such as methyl laurate, ethyl laurate, butyl laurate, methyl myristate, ethyl myristate, butyl myristate, methyl palmitate, ethyl palmitate, and butyl palmitate. Since fatty acid esters are liquid at room temperature, have a good dispersion effect, and have characteristics similar to those of alkanes, they can replace the substrate alkanes used at the present stage. Fatty acid esters mainly come from the seeds of oil-rich tropical crops such as palm trees and coconut trees. This novel renewable resource can replace fossil raw materials such as petroleum alkanes, which is of great significance for the sustainable development of industrial processes.

The present disclosure does not particularly limit the expansion culture mode of the fermentation strain, for example, a conventional mode of slant strain-shake flask activation followed by seed culture (seed tank) is adopted.

In some embodiments, in step (1), the expansion culture performed on the fermentation strain comprises: subjecting the fermentation strain to an activation culture in a shake flask, and when the shake flask seed has an optical density value OD₆₂₀ of 0.5-1.0 after a 30-fold dilution, inoculating the shake flask seed into a seed tank containing a seed culture medium for seed culture until the obtained seed liquid has an optical density value OD₆₂₀ of 0.5-1.0 after a 30-fold dilution.

In some embodiments, conditions for the activation culture comprise: a temperature of 27-35°C, a shaker rotation speed of 150-250 rpm, and a time of 38-42 h.

In some embodiments, in step (1), the seed culture medium comprises a nitrogen source, a carbon source, an inorganic salt, a growth factor, a defoamer, and the like; the carbon source includes, but is not limited to, glucose, maltose, sucrose, and the like; the nitrogen source includes, but is not limited to, corn steep liquor, urea, yeast extract, potassium nitrate, ammonium sulfate, and the like; the inorganic salt includes, but is not limited to, potassium dihydrogen phosphate, dipotassium hydrogen phosphate, sodium dihydrogen phosphate, potassium nitrate, sodium chloride, ferrous sulfate, magnesium sulfate, and the like; the nutrition factor includes, but is not limited to, vitamin B1, vitamin B2, vitamin B5, biotin, and the like.

In some embodiments, the seed culture medium comprises the following components: 0.5%-3.5% of sucrose, 0.2%-0.8% of corn steep liquor, 0.1%-1% of yeast extract, 0.2%-1.0% of potassium dihydrogen phosphate, 0.2%-0.8% of urea, and 0.02%-0.10% of a defoamer.

The percentages used to characterize component contents in the culture medium of the present disclosure all refer to general conventions in the field of fermentation, and the percentages represent weight-to-volume ratios (w/v), i.e., % represents g/100 mL.

In some embodiments, during the seed culture, a temperature is controlled at 27-35°C, an aeration rate is controlled at 0.2-0.8 vvm, a pressure is controlled at 0.05-0.20 MPa, and an amount of dissolved oxygen is controlled at 5%-50%.

In some embodiments, in step (2), the seed liquid is inoculated into a fermentation culture medium at an inoculation amount of 10-50 v/v% to perform fermentation, that is, a volume ratio of the seed liquid to the fermentation culture medium is (10-50):100.

In some embodiments, the fermentation is performed in a fermentation tank, and the fermentation culture medium comprises a nitrogen source, a carbon source, a growth factor, an inorganic salt, and the like.

In some embodiments, the fermentation culture medium comprises the following components: 0%-1.0% of corn steep liquor, 2.0%-6.0% of glucose, 0%-0.8% of yeast extract, 0.4%-1.0% of potassium dihydrogen phosphate, 0.1%-0.6% of magnesium sulfate, 0.1%-1% of ammonium sulfate, and 0.2%-0.8% of potassium nitrate.

In some embodiments, in step (2), when the fermentation broth has an optical density value OD₆₂₀ of 0.5-1.0, preferably 0.7-1.0, after a 30-fold dilution, a fatty acid ester is added throughout the process.

In some embodiments, in step (2), an addition mode of the fatty acid ester comprises: adding the fatty acid ester in a mode of one-time addition, batch supplementation, or continuous feeding, and from the start of fermentation to T h, a viscosity η and a fermentation time t of the fermentation system are controlled to satisfy η = ae^{bt} + c by supplementing the fatty acid ester, wherein 1.5 ≤ a ≤ 5.5, 0.01 ≤ b ≤ 0.05, 0 ≤ c ≤ 10, and 135 ≤ T ≤ 165; the unit of the viscosity η is mPa·s, and the unit of the fermentation time **t** is h.

In some embodiments, the numerical range of b satisfies 0.02 ≤ b ≤ 0.05.

In some embodiments, the numerical range of c satisfies 0 ≤ c ≤ 6.

It should be noted that e is a natural constant, a constant in mathematics, an infinite non-repeating decimal, and a transcendental number with a value of about 2.718281828459045.

Those skilled in the art will appreciate that the fermentation time t is in the range of greater than 0 and less than or equal to T.

In one embodiment, the supplementation of the fermentation substrate is stopped after T h of fermentation. Specifically, after the supplementation of the fatty acid ester is stopped, the fermentation is performed by utilizing the residual fatty acid ester in the fermentation system until the fatty acid ester is completely consumed or the long-chain dicarboxylic acid is no longer produced, and the total fermentation time is calculated.

In some embodiments, in step (2), during the fermentation, a temperature is controlled at 27-33°C, an aeration rate is controlled at 0.2-0.8 vvm, a pressure is controlled at 0.05-0.20 MPa, a pH value is controlled at 4.5-6.5, an amount of dissolved oxygen is controlled at 5%-50%, and the fermentation is performed for 120-200 h. Specifically, during the fermentation, the pH value of the fermentation broth can be maintained at 4.5-6.5, preferably 5.5-6.5, by adding a small amount of an alkaline substance such as sodium hydroxide.

In some embodiments, in step (3), the purification comprises: acidifying the long-chain dicarboxylic acid fermentation broth, performing separation to obtain a solid matter, then dissolving the solid matter in an organic solvent, performing separation to obtain a clear liquid, and performing crystallization to obtain a long-chain dicarboxylic acid product.

In one embodiment, the method comprises: subjecting a fermentation broth to a purification treatment, wherein the purification treatment comprises: acidifying the fermentation broth, performing separation to obtain a solid matter, then dissolving the solid matter in an organic solvent, subjecting the mixture to crystallization and solid-liquid separation, and subjecting the separated solid to a hot water treatment, washing, and drying to obtain a long-chain dicarboxylic acid product.

In some embodiments, the acidification is performed at a pH of 2-5, preferably 3.5-4.5.

In some embodiments, a method of the solid-liquid separation described above comprises at least one of filtration or centrifugation.

In some embodiments, the organic solvent described above comprises one or more of an acid, an alcohol, an ester, a ketone, and the like, wherein the alcohol comprises one or more of methanol, ethanol, n-butanol, and isopropanol, the acid comprises acetic acid, the ketone comprises acetone, and the ester comprises at least one of ethyl acetate and butyl acetate.

In some embodiments, decolorization is performed after dissolving in the organic solvent, and then a clear liquid is obtained by separation. A method of the decolorization is preferably activated carbon decolorization, the decolorization is performed at a temperature of 80-110°C, and the decolorization is performed at a time of 30-190 min.

In one embodiment, an addition amount of a decolorizing agent during the decolorization treatment is 0.5%-10%, preferably 1%-5%, and further preferably 1.2%-4.8% of the mass of the long-chain dicarboxylic acid contained in the liquid to be decolorized.

In one embodiment, the hot water treatment is performed at a temperature of 70-150°C, preferably 70-135°C.

In one embodiment, the hot water treatment comprises the following step: contacting the separated solid with hot water to remove an impurity, wherein a temperature of the hot water is 70-150°C, preferably 70-135°C.

In one embodiment, the hot water treatment comprises the following steps: mixing the solid with water in a mass ratio of 1:(1-10), incubating at 70-150°C for 30-180 min, cooling to 25-60°C, and performing solid-liquid separation to obtain a solid.

In one embodiment, the hot water treatment comprises the following steps: mixing the solid with water in a mass ratio of 1:(1-10), incubating at 70-135°C for 30-180 min, cooling to 25-60°C, and performing solid-liquid separation to obtain a solid.

In one embodiment, the solid obtained after the hot water treatment is washed and dried.

In some embodiments, the crystallization is cooling crystallization, and the cooling crystallization comprises the following steps: cooling to 50-80°C, incubating for 2 h, cooling to 20-40°C, and finally crystallizing.

In some embodiments, a long-chain dicarboxylic acid product is obtained by separation after the crystallization.

In a second aspect of the present disclosure, the present disclosure provides a long-chain dicarboxylic acid, and the long-chain dicarboxylic acid has a purity of 98% or higher, preferably 99% or higher, and a bio-based content of 100%.

### DETAILED DESCRIPTION OF THE INVENTION

To make the objectives, technical solutions, and advantages of the examples of the present disclosure clearer, the technical solutions in the examples of the present disclosure will be clearly and completely described below. Apparently, the described examples are only a part rather than all of the examples of the present disclosure. All other examples obtained by those of ordinary skill in the art, based on the examples in the present disclosure, without making creative efforts, shall fall within the protection scope of the present disclosure.

Fermentation strain used in the following examples: *Candida viswanathii* CAES2113, which has been disclosed in CN111748480A, was biologically deposited on Feb. 24, 2020, at the China Center for Type Culture Collection (address: Wuhan University, Wuhan, China), with an accession number of CCTCC M 2020048, and a taxonomic name of *Candida viswanathii*.

In the following examples, the content of the long-chain dicarboxylic acid in the fermentation broth is determined by gas chromatography (GC). Specifically, the fermentation broth is treated and then separated in a capillary column, and the content of the dicarboxylic acid in the fermentation broth is determined according to the peak area of the compound.

Determination method for bio-based content: determined by measuring the content of radioactive C14, for example, the standard ASTM-D6866 method of the American Society for Testing and Materials.

### Example 1

(1) First, the pH value of a wort of 10°Bé was adjusted to about 5.4, and then 100 mL of the wort was placed in a conical flask and sterilized at 121°C for 20 min. Subsequently, 1 tube of seeds of *Candida viswanathii* preserved in a 2 mL glycerol tube stored in a -80°C refrigerator in advance was inoculated, and activation culture was performed on a rotary shaker at a temperature of 29°C and a rotation speed of 220 rpm for 38-42 h. When the OD₆₂₀ value of the culture reached 0.8 (30-fold dilution), the activation culture was terminated.
(2) The shake flask seeds obtained in step (1) described above were inoculated into a seed tank containing a seed culture medium at an inoculation amount of 1.5% and cultured, and during the culture, the temperature was controlled at 29°C, the aeration rate was controlled at 0.3 vvm, the pressure was controlled at 0.10 MPa, and the amount of dissolved oxygen during the fermentation was maintained at 30%, until the OD₆₂₀ value of the obtained seed liquid reached 0.5-1.0 after a 30-fold dilution.

Components of the seed culture medium were: 0.5% of corn steep liquor, 2.3% of sucrose, 0.3% of urea, 0.5% of yeast extract, 0.6% of potassium dihydrogen phosphate, and 0.05% of a defoamer.

(3) The seed liquid obtained in step (2) described above was inoculated into a fermentation tank containing a fermentation culture medium to perform fermentation, with an inoculation amount of 15% (v/v) and a fermentation temperature of 29°C, and the pH value during the fermentation is controlled at 6.4, the aeration rate is controlled at 0.3 vvm, the pressure during the fermentation is controlled at 0.10 MPa, and the amount of dissolved oxygen during the fermentation is maintained at 40%.

The fermentation culture medium in the fermentation tank comprises the following components: 0.5% of potassium dihydrogen phosphate, 0.8% of corn steep liquor, 3.5% of glucose, 0.4% of ammonium sulfate, 0.4% of yeast extract, 0.5% of potassium nitrate, and 0.2% of magnesium sulfate.

After the seed liquid was inoculated into the fermentation tank, when the OD₆₂₀ value of the culture reached 0.8 (30-fold dilution), the substrate laurate was fed into the fermentation tank to perform fermentation and conversion. During the fermentation, the viscosity η of the fermentation system was monitored by an online viscosity detector, and the supplementation amount of methyl laurate was controlled such that the viscosity η and the fermentation time t of the fermentation system satisfied η = ae^{bt} + c, wherein a = 5.10, b = 0.035, and c = 1.5. After 150 h of fermentation, the feeding of the substrate was stopped, the substrate was consumed completely, and the fermentation was completed.

### Example 2

The substrate was replaced with ethyl laurate, and the remaining procedures were the same as those in Example 1.

### Example 3

The substrate was replaced with butyl laurate, and the remaining procedures were the same as those in Example 1.

### Example 4

The supplementation amount of methyl laurate was controlled such that the viscosity η and the fermentation time t of the fermentation system satisfied η = ae^{bt} + c, wherein the unit of the viscosity η is mPa·s, wherein a = 1.60, b = 0.03, and c = 5; the remaining procedures were the same as those in Example 1.

### Example 5

The supplementation amount of methyl laurate was controlled such that the viscosity η and the fermentation time t of the fermentation system satisfied η = ae^{bt} + c, wherein the unit of the viscosity η is mPa·s, wherein a = 1.50, b = 0.05, and c = 5.1; the remaining procedures were the same as those in Example 1.

### Example 6

The substrate was replaced with methyl decanoate, and the supplementation amount of the methyl decanoate was controlled such that the viscosity η and the fermentation time t of the fermentation system satisfied η = ae^{bt} + c, wherein the unit of the viscosity η is mPa·s, wherein a = 5.40, b = 0.022, and c = 1.2; the remaining procedures were the same as those in Example 1.

### Example 7

The substrate was replaced with ethyl tetradecanoate, and the supplementation amount of the ethyl tetradecanoate was controlled such that the viscosity η and the fermentation time t of the fermentation system satisfied η = ae^{bt} + c, wherein the unit of the viscosity η is mPa·s, wherein a = 3.50, b = 0.038, and c = 3.1; the remaining procedures were the same as those in Example 1.

### Comparative Example 1

The supplementation amount of methyl laurate was controlled such that the viscosity η and the fermentation time t of the fermentation system satisfied η = ae^{bt} + c, wherein a = 6.60, b = 0.06, and c = 0; the remaining procedures were the same as those in Example 1.

The fermentation results of Examples 1-7 and Comparative Example 1 are shown in Table 1:

**Table 1**

| No. | Substrate type | Acid yield/(g/L) | Conversion rate/wt% | Total fermentation time/h |
|---|---|---|---|---|
| Example 1 | Methyl laurate | 169 | 81.7 | 160 |
| Example 2 | Ethyl laurate | 163 | 77.5 | 165 |
| Example 3 | Butyl laurate | 136 | 68.2 | 170 |
| Example 4 | Methyl laurate | 162 | 80.1 | 161 |
| Example 5 | Methyl laurate | 163 | 79.2 | 161 |
| Example 6 | Methyl decanoate | 154 | 77.2 | 164 |
| Example 7 | Ethyl tetradecanoate | 160 | 75.2 | 161 |
| Comparative Example 1 | Methyl laurate | 159 | 78.5 | 162 |

The fermentation broths obtained in Examples 1-7 and Comparative Example 1 above were each separated and purified, specifically as follows: sulfuric acid was added to the fermentation broth to adjust the pH to 3-4, followed by acidification and crystallization, and separation was performed to obtain a solid matter. This step effectively removed bacterial cells, fatty acid esters, and metabolic impurities affecting downstream polymerization, such as fatty acids and hydroxy fatty acids, in the fermentation broth, thus obtaining a crude product of a long-chain dicarboxylic acid.

The crude product of the long-chain dicarboxylic acid described above was placed into a decolorization tank, and acetic acid was added, with the ratio of the crude product of the long-chain dicarboxylic acid to the acetic acid being controlled at 1:(3-6). Activated carbon was added, and the addition amount of the activated carbon was 0.5%-10% of the mass of the dodecanedioic acid in the liquid to be decolorized. The above materials were heated to 90-110°C and decolorized for 30-180 min, and a clear liquid of the long-chain dicarboxylic acid was obtained through filtration by a plate and frame filter press. The obtained clear liquid was placed into a crystallization tank, cooled to 30-80°C, incubated for 2-3 h, and then cooled to 10-30°C. After crystals were precipitated, solid-liquid separation was performed by a centrifuge. The obtained crystal was added to water, and the mass ratio of the solid to the water was 1:(1-10). After 30-180 min of incubation at 70-135°C, the system was cooled to 25-60°C, and filtration was performed to obtain a solid. The solid was washed, washed with water, and dried to obtain a long-chain dicarboxylic acid product.

Upon further testing, the content of the long-chain dicarboxylic acid in the long-chain dicarboxylic acid product was no less than 99.5%. Taking the separation and purification of the fermentation broth obtained in Example 1 as an example, the obtained long-chain dicarboxylic acid product had a long-chain dicarboxylic acid (DC12) content of 99.8% and a bio-based content of 100%.

Finally, it should be noted that: the above examples are only used to illustrate the technical solutions of the present disclosure, rather than to limit them; although the present disclosure has been described in detail with reference to the foregoing examples, those of ordinary skill in the art should understand that: modifications can still be made to the technical solutions recorded in the foregoing examples, or equivalent replacements can be made to some or all of the technical features therein; and these modifications or replacements do not make the essence of corresponding technical solutions depart from the scope of the technical solutions of the examples of the present disclosure.

## Claims

1. A method for producing a long-chain dicarboxylic acid, comprising: inoculating a seed liquid of a fermentation strain into a fermentation culture medium and culturing, and adding a substrate fatty acid ester to perform fermentation.

2. The method according to claim 1, comprising:
(1) subjecting the fermentation strain to expansion culture until the seed liquid of the fermentation strain has an optical density value OD₆₂₀ of 0.5-1.0 after a 30-fold dilution;
(2) inoculating the seed liquid into a fermentation culture medium, and when the fermentation broth has an optical density value OD₆₂₀ of 0.5-1.0 after a 30-fold dilution, starting supplementing the fatty acid ester to obtain a long-chain dicarboxylic acid fermentation broth; and
(3) purifying the long-chain dicarboxylic acid fermentation broth to obtain a long-chain dicarboxylic acid.

3. The method according to claim 1, wherein the long-chain dicarboxylic acid has a chemical formula of HOOC(CH₂)nCOOH, wherein n ≥ 8; the long-chain dicarboxylic acid comprises any one of decanedioic acid, undecanedioic acid, dodecanedioic acid, tridecanedioic acid, tetradecanedioic acid, pentadecanedioic acid, hexadecanedioic acid, heptadecanedioic acid, and octadecanedioic acid; and/or,
the carbon source comprises any one or two of glucose, fructose, sucrose, and maltose; and/or,
the fermentation strain comprises *Candida viswanathii*, *Candida albicans*, *Candida tropicalis*, *Candida sake*, or *Yarrowia lipolytica*; and/or,
the fatty acid ester comprises any one of a decanoate, an undecanoate, a laurate, a tridecanoate, a myristate, a pentadecanoate, a palmitate, a heptadecanoate, and an octadecanoate.

4. The method according to claim 1, wherein an addition mode of the fatty acid ester comprises: adding the fatty acid ester in a mode of batch supplementation or continuous feeding, and from the start of fermentation to T h, a viscosity η and a fermentation time t of the fermentation system are controlled to satisfy η = ae^{bt} + c by supplementing the fatty acid ester, wherein 1.5 ≤ a ≤ 5.5, 0.01 ≤ b ≤ 0.05, 0 ≤ c ≤ 10, and 135 ≤ T ≤ 165; e is a natural constant, the unit of the viscosity η is mPa·s, and the unit of the fermentation time **t** is h.

5. The method according to claim 2, wherein in step (1), the expansion culture performed on the fermentation strain comprises:
subjecting the fermentation strain to an activation culture in a shake flask, and when the shake flask seed has an optical density value OD₆₂₀ of 0.5-1.0 after a 30-fold dilution, inoculating the shake flask seed into a seed tank containing a seed culture medium for seed culture until the obtained seed liquid has an optical density value OD₆₂₀ of 0.5-1.0 after a 30-fold dilution.

6. The method according to claim 5, wherein the seed culture medium comprises the following components: 0.5%-3.5% of sucrose, 0.2%-0.8% of corn steep liquor, 0.1%-1% of yeast extract, 0.2%-1.0% of potassium dihydrogen phosphate, 0.2%-0.8% of urea, and 0.02%-0.10% of a defoamer; and/or,
during the seed culture, a temperature is controlled at 27-35°C, an aeration rate is controlled at 0.2-0.8 vvm, a pressure is controlled at 0.05-0.20 MPa, and an amount of dissolved oxygen is controlled at 5%-50%.

7. The method according to claim 2, wherein the fermentation culture medium comprises the following components: 0%-1.0% of corn steep liquor, 2.0%-6.0% of glucose, 0%-0.8% of yeast extract, 0.4%-1.0% of potassium dihydrogen phosphate, 0.1%-0.6% of magnesium sulfate, 0.1%-1% of ammonium sulfate, and 0.2%-0.8% of potassium nitrate; and/or,
the seed liquid is inoculated into a fermentation culture medium at an inoculation amount of 10-50 v/v% to perform fermentation; and/or,
during the fermentation, a temperature is controlled at 27-33°C, an aeration rate is controlled at 0.2-0.8 vvm, a pressure is controlled at 0.05-0.20 MPa, a pH value is controlled at 4.5-6.5, the fermentation is performed for 120-200 h, and an amount of dissolved oxygen is controlled at 5%-50%.

8. The method according to claim 2, wherein the purification comprises: acidifying the long-chain dicarboxylic acid fermentation broth, performing separation to obtain a solid matter, then dissolving the solid matter in an organic solvent, performing separation to obtain a clear liquid, and performing crystallization to obtain a long-chain dicarboxylic acid product.

9. The method according to claim 2, wherein the purification comprises: acidifying the fermentation broth, performing separation to obtain a solid matter, then dissolving the solid matter in an organic solvent, subjecting the mixture to crystallization and solid-liquid separation, and subjecting the separated solid to a hot water treatment, washing, and drying to obtain a long-chain dicarboxylic acid product.

10. The method according to claim 9, wherein the hot water treatment is performed at a temperature of 70-150°C.

11. The method according to claim 9, wherein the hot water treatment comprises the following steps: mixing the solid with water in a mass ratio of 1:(1-10), incubating at 70-150°C for 30-180 min, cooling to 25-60°C, and performing solid-liquid separation to obtain a solid.

12. A long-chain dicarboxylic acid, wherein the long-chain dicarboxylic acid has a purity of 98% or higher, preferably 99% or higher, and a bio-based content of 100%.
